# EUROPEAN PATENT APPLICATION

(11) **EP 4 793 807 A1**
(43) Date of publication of application: **19.08.2026**
(21) Application number: 25157517.1
(22) Date of filing: 12.02.2025
(51) Int. Cl.: G06F 21/62, G16H 10/00, G16H 40/00, G16H 80/00, H04W 4/38, H04W 4/80, H04W 12/02

(54) **ANONYMOUS SECURE DATA CAPTURE FROM ASSISTIVE DEVICE WORN BY A USER**

(71) Applicant: Blatchford Products Limited, Basingstoke, Hampshire RG24 8PZ (GB)
(72) Inventor: Odeyale, Akinkunmi, Northampton, NN3 5ET (GB); Woodhead, Robert Paul, Warwick, CV34 5NS (GB)
(74) Representative: Hepworth Browne

(57) **Abstract**

Embodiments herein provide a method and system for anonymous secure data capture from an assistive device (103) worn by a user (108). The method includes receiving by mobile device (101), a recording message with an access link, a usage permission indicating number of permissible accesses, and a validity duration for the access link, from a central server (102) over a first mode of communication. The mobile device (101) displays the access link on user interface. On detection of the user interaction, the mobile device (101) establishes a secure wireless connection between the mobile device (101) and assistive device (103). The mobile device (101) captures the measurement data from the sensors associated with the assistive device (103). Simultaneously, the mobile device (101) captures video data of the user (108) wearing the assistive device (103). The mobile device (101) transmits the recorded video and the measurement data to the central server (102).

## Description

### FIELD OF INVENTION

The proposed embodiments relate to medical device system and more particularly relates to a method and system for anonymous secure data capture from assistive device worn by a user.

### BACKGROUND

Prosthetic and orthotic devices play a crucial role in modern healthcare by providing support, protection, and functional restoration to individuals affected by injuries, disabilities, or congenital conditions. These devices are tailored to meet the specific needs of patient, aiming to enhance mobility, alleviate pain, and restore functionality. Prosthetics are designed to replace missing body parts, offering advanced capabilities to help individuals regain independence and mobility. Orthotic devices provide stabilization and support to joints, assisting those with musculoskeletal issues or in need of rehabilitation. As such, both prosthetics and orthotics are indispensable tools that significantly improve the quality of life for individuals facing physical challenges.

In recent years, remote monitoring technologies have become increasingly integrated into medical devices, including prosthetics and orthotics. These technologies enable continuous tracking of a patient's health and device status without necessitating frequent in-person visits. The benefits of remote monitoring are manifold, including the continuous collection of data, timely interventions, and improved management of chronic conditions. Prosthetic and orthotic devices equipped with sensors and remote monitoring systems can collect valuable health information, such as gait patterns, pressure points, movement data, and physiological measurements. This information is invaluable to healthcare providers for making informed clinical decisions and optimizing treatment plans.

However, the integration of remote monitoring technologies in prosthetic and orthotic devices presents several challenges, particularly concerning data security and patient privacy. The transmission of sensitive health data from these devices is vulnerable to interception, hacking, and data breaches. Cyberattacks pose a significant risk, as attackers could potentially alter or manipulate the data being transmitted, leading to incorrect clinical decisions based on inaccurate information. Such tampering could result in inappropriate adjustments or treatments, potentially compromising patient safety and outcomes.

Moreover, the continuous monitoring of movement, pressure, and device wear could inadvertently reveal private details about a patient's daily activities or behavior. Without robust encryption, secure data storage, and clear patient consent protocols, these devices risk infringing on patient privacy rights. The exposure of sensitive health information, such as gait patterns and patient history, underscores the need for stringent cybersecurity measures to protect against unauthorized access and misuse.

Existing systems that facilitate data retrieval or transmission from sensor-based prosthetic or orthotic devices often utilize Bluetooth Low Energy (BLE) technology. While BLE offers a convenient means of connectivity, it also introduces security vulnerabilities. Users must be authorized to connect to the device, and while they can access their data as part of the device's features, there remains a risk of data interception for malicious purposes. If a mobile device used for data access is compromised through hacking or faulty software, it can lead to incorrect readings, false diagnoses, or even life-threatening situations.

Given these challenges, there is a clear need to address the security and privacy concerns associated with remote monitoring technologies in prosthetic and orthotic devices. Solutions that enhance data protection, ensure patient privacy, and maintain the integrity of transmitted information are essential to fully realize the potential benefits of these advanced medical devices.

### SUMMARY

In an aspect, the objects are achieved by providing a method for anonymous secure data capture from an assistive device worn by a user. The method includes receiving by a mobile device a recording message for anonymous secure data capture from a central server over a first mode of communication. The recording message includes an access link that identifies the assistive device to be recorded, a usage permission indicating the number of permissible accesses, and a validity duration for the access link. The mobile device displays the access link on the user interface of the mobile device. Further, the mobile device detects the user interaction with the access link displayed on the user interface of the mobile device. The mobile device further establishes a secure wireless connection between the mobile device and the assistive device worn by the user using a second mode of communication different from the first mode of communication. The secure wireless connection between the mobile device and the assistive device is established based on the access link, the usage permission, and the validity duration. The mobile device captures the measurement data from the sensors embedded in the assistive device based on the secure wireless connection. Simultaneously, the mobile device records video data of the user wearing the assistive device while capturing the measurement data from the plurality of sensors embedded in the assistive device. Further, the mobile device transmits the video data of the user wearing the assistive device and the measurement data from the sensors embedded in the assistive device to the central server.

In another aspect, the objects are achieved by providing a system for anonymous secure data capture from the assistive device worn by the user. The system includes a central server, the mobile device, and the assistive device worn by the user. The mobile device further includes the memory, an application, an anonymous secure data controller connected to the memory. The anonymous secure data controller receives a recording message for anonymous secure data capture from a central server over a first mode of communication. The recording message comprises an access link that identifies the assistive device to be recorded, a usage permission indicating the number of permissible accesses, and a validity duration for the access link. The anonymous secure data controller displays the access link on a user interface of the mobile device of the user. Further, the anonymous secure data controller detects a user interaction with the access link displayed on the user interface of the mobile device. In response to the user interaction, the anonymous secure data controller establishes a secure wireless connection between the mobile device and the assistive device worn by the user using a second mode of communication different from the first mode of communication. The secure wireless connection between the mobile device and the assistive device is established based on the access link, the usage permission, and the validity duration. Further, the anonymous secure data controller captures measurement data from a plurality of sensors embedded in the assistive device based on the secure wireless connection and simultaneously records the video data of the user wearing the assistive device while capturing the measurement data from the plurality of sensors embedded in the assistive device. Furthermore, the anonymous secure data controller transmits the video data of the user wearing the assistive device and the measurement data from the plurality of sensors embedded in the assistive device to the central server.

These and other aspects of the embodiments herein will be better appreciated and understood when considered in conjunction with the following description and the accompanying drawings. It is understood, however, that the following descriptions, while indicating preferred embodiments and numerous specific details thereof, are given by way of illustration and not of limitation. Many changes and modifications be made within the scope of the embodiments herein without departing from the spirit thereof, and the embodiments herein include all such modifications.

### BRIEF DESCRIPTION OF FIGURES

These and other features, aspects, and advantages of the present embodiments are illustrated in the accompanying drawings, throughout which like reference letters indicate corresponding parts in the various figures. The embodiments herein will be better understood from the following description with reference to the drawings, in which:

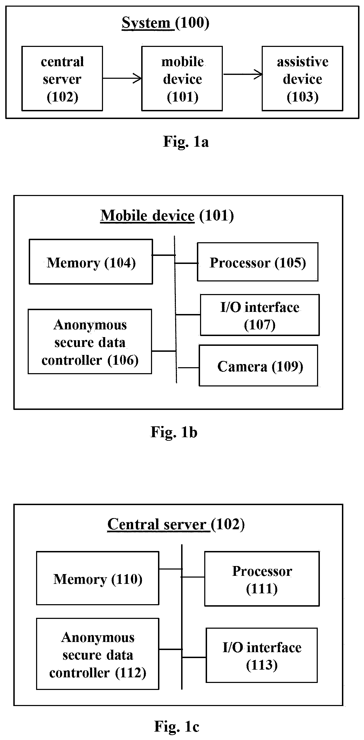

Fig. 1a is a block diagram that illustrates the hardware features of the system for anonymous secure data capture according to the embodiments as disclosed herein. Fig. 1b is a block diagram that illustrates the hardware features of the mobile device according to the embodiments as disclosed herein. Fig. 1c is a block diagram that illustrates the hardware features of the central server, according to the embodiments as disclosed herein.

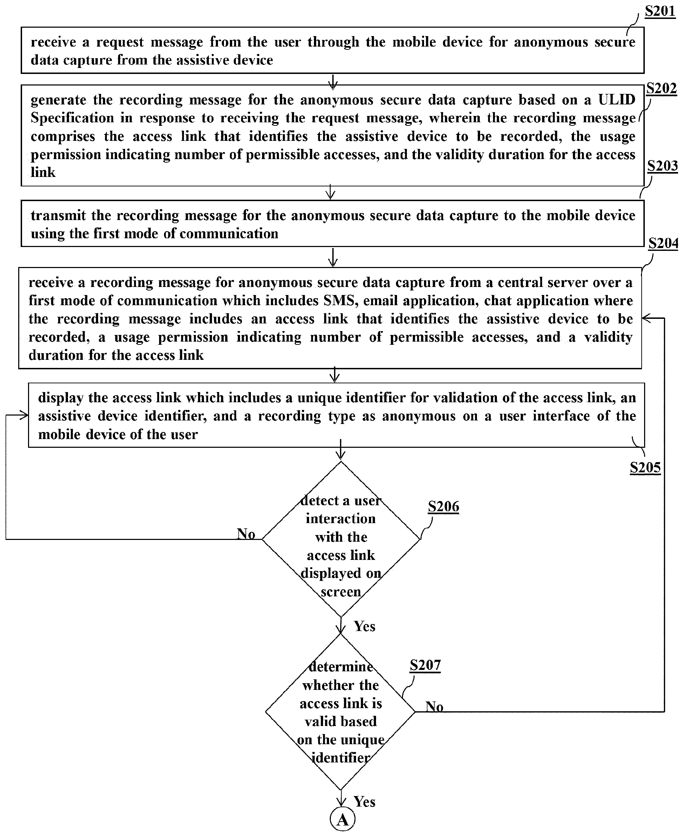

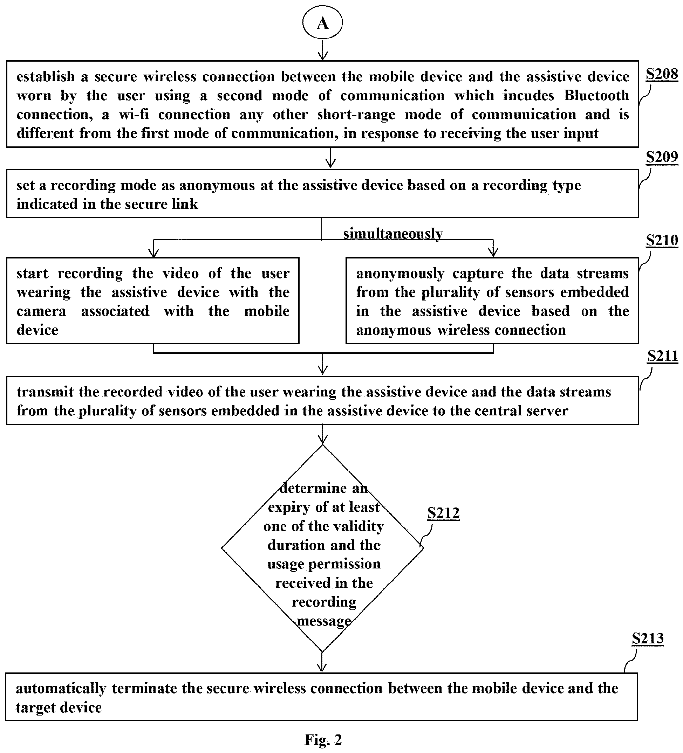

FIG. 2 is a flow diagram that illustrates a method of anonymous secure data capture from an assistive device worn by a user according to the embodiments as disclosed herein.

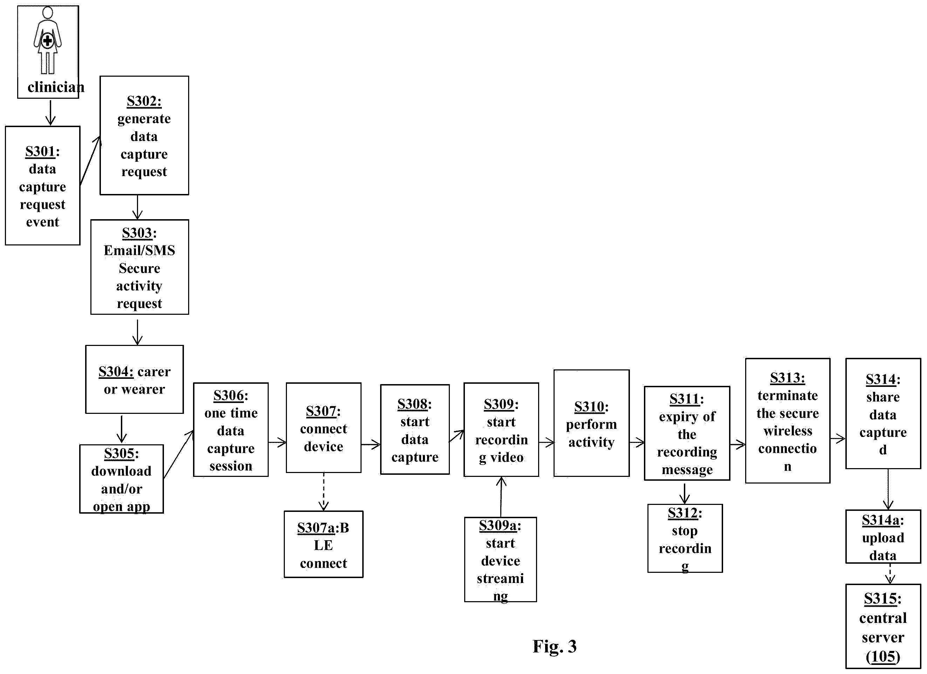

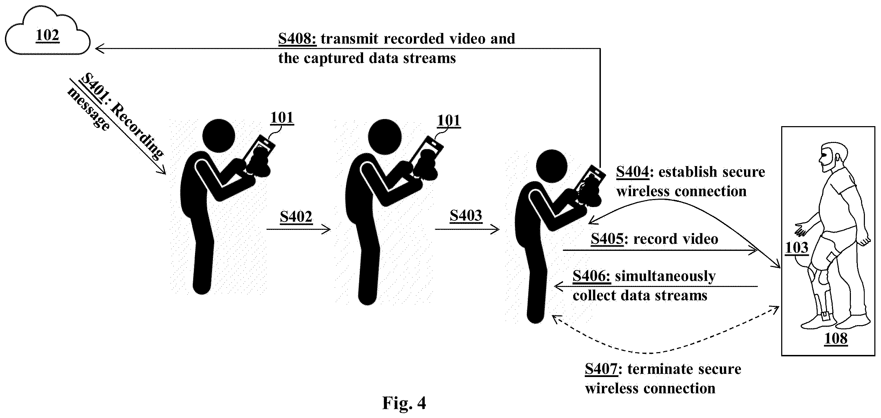

Fig. 3 illustrates a use case scenario of capturing the anonymous secure data from the assistive devices according to the embodiments as disclosed herein. Fig. 4 illustrates a use case scenario of capturing the anonymous secure data from the prosthetic device worn by a user according to the embodiments as disclosed herein.

It is noted that to the extent possible, like reference numerals have been used to represent like elements in the drawing. Further, those of ordinary skill in the art will appreciate that elements in the drawing are illustrated for simplicity and may not have been necessarily drawn to scale. For example, the dimension of some of the elements in the drawing is exaggerated relative to other elements to help to improve the understanding of aspects of the invention. Furthermore, the elements may have been represented in the drawing by existing symbols, and the drawings may show only those specific details that are pertinent to the understanding the embodiments of the invention so as not to obscure the drawing with details that will be readily apparent to those of ordinary skill in the art having benefit of the description herein.

### DETAILED DESCRIPTION OF INVENTION

The embodiments herein and the various features and advantageous details thereof are explained more fully with reference to the non-limiting embodiments that are illustrated in the accompanying drawings and details in the following description. Descriptions of well-known components and processing techniques are omitted so as to not unnecessarily obscure the embodiments herein. Also, the various embodiments described herein are not necessarily mutually exclusive, as some embodiments can be combined with one or more other embodiments to form new embodiments. The term "or" as used herein, refers to a non-exclusive or, unless otherwise indicated. The examples used herein are intended merely to facilitate an understanding of ways in which the embodiments herein can be practiced and to further enable those skilled in the art to practice the embodiments herein. Accordingly, the examples are not be construed as limiting the scope of the embodiments herein.

As is traditional in the field, embodiments are described and illustrated in terms of blocks that carry out a described function or functions. These blocks, which referred to herein as managers, units, modules, hardware components or the like, are physically implemented by analog and/or digital circuits such as logic gates, integrated circuits, microprocessors, microcontrollers, memory circuits, passive electronic components, active electronic components, optical components, hardwired circuits and the like, and optionally be driven by firmware and software. The circuits, for example, be embodied in one or more semiconductor chips, or on substrate supports such as printed circuit boards and the like. The circuits constituting a block be implemented by dedicated hardware, or by a processor (e.g., one or more programmed microprocessors and associated circuitry), or by a combination of dedicated hardware to perform some functions of the block and a processor to perform other functions of the block. Each block of the embodiments be physically separated into two or more interacting and discrete blocks without departing from the scope of the proposed method. Likewise, the blocks of the embodiments be physically combined into more complex blocks without departing from the scope of the proposed method.

The accompanying drawings are used to help easily understand various technical features and it is understood that the embodiments presented herein are not limited by the accompanying drawings. As such, the proposed method is construed to extend to any alterations, equivalents and substitutes in addition to those which are particularly set out in the accompanying drawings. Although the terms first, second, etc. used herein to describe various elements, these elements are not be limited by these terms. These terms are generally used to distinguish one element from another.

Modern prosthetic and orthotic devices, including robotic limbs and arms, smart braces are becoming increasingly sophisticated by integrating computational communication and control mechanisms to monitor and improve user performance. These devices gather physiological data, such as muscle activity and joint angles, and communicate it to users or healthcare providers. However, as these devices offer enhanced functionality, they also present new security vulnerabilities. Mobile devices connected to prosthetic and orthotic devices are particularly at risk, as hackers can exploit weaknesses in communication protocols, intercept or alter data, and manipulate device controls. Additionally, the permanent nature of data links in existing systems poses a significant security threat, allowing sensitive information to be accessed, shared, or compromised indefinitely.

In contrast to the existing methods, the present solution provides a anonymous secure data capture from the assistive devices worn by the user. The system and the method includes receiving by the mobile device, a recording message with an access link, a usage permission indicating number of permissible accesses, and a validity duration for the access link, from a central server over a first mode of communication. The mobile device displays the access link on the user interface. On detection of the user interaction, the mobile device establishes a secure wireless connection between the mobile device and the assistive device. The mobile device captures the measurement data from the sensors associated with the assistive device. Simultaneously, the mobile device captures video data of the user wearing the assistive device. The mobile device transmits the recorded video and the measurement data to the central server.

Referring now to the drawings and more particularly to Figs. 1 through 4, where similar reference characters denote corresponding features consistently throughout the figure, these are shown preferred embodiments.

Fig 1a is a block diagram that illustrates the hardware features of the system (100) for anonymous secure data capture according to the embodiments as disclosed herein. In an embodiment, the system (100) includes a central server (102), a mobile device (101), and an assistive device (103). The system (100) is designed to ensure that data captured from users remains anonymous and secure, leveraging advanced encryption protocols and anonymization techniques. The architecture is modular, allowing for easy integration of additional devices or sensors as needed, and is scalable to accommodate a growing number of users or data points. The system's design also considers energy efficiency, with components optimized for low power consumption to extend the operational life of mobile and assistive devices.

The central server (102) manages and stores data, processes requests, and provides services to other devices or users in a network. The central server (102) stores and manages files, databases, and other information for access by multiple users or devices. Further, the central server (102) handles requests from client devices and processes or sends back the appropriate responses. It serves as the core point for managing network resources, ensuring efficient communication and consistency across connected devices. The central server (102) is equipped with high-performance processors and large storage capacities to handle vast amounts of data and complex computations. It also includes redundant systems and failover mechanisms to ensure high availability and reliability, even in the event of hardware failures or network disruptions.

In an embodiment, the cloud-based platform or the central server (102) serves as a central repository for storing and managing collected data. This centralized approach enables secure data backup, efficient data sharing, and collaboration among healthcare providers. The platform employs robust access control mechanisms to ensure that only authorized users can access sensitive data. Additionally, the platform facilitates large-scale data analysis and the development of predictive models or generative Artificial Intelligence (AI) models to identify potential abnormalities or treatment outcomes. These models can be used to simulate various intervention scenarios, providing clinicians with evidence-based recommendations for optimizing patient care and improving outcomes. The platform's AI capabilities are enhanced by machine learning techniques that continuously learn from new data, improving the accuracy and reliability of predictions over time.

In an embodiment, the mobile device (101) includes but is not limited to mobile devices, smartphones, laptops, digital cameras, desktops, Personal Digital Assistants (PDAs), and tablets. In an embodiment, the mobile device (101) may belong to the user (108), the care taker, the clinician or any other person responsible for taking care of the user of the assistive device (103).

The mobile device (101) handles receiving the recording message from the central server (102), displaying the access link on a user interface of the mobile device (101), and detecting user interaction with the access link displayed on the user interface. Further, the mobile device (101) establishes, in response to receiving the user interaction, a secure wireless connection between the mobile device (101) and the assistive device (103) worn by the user using a second mode of communication different from the first mode of communication. The secure wireless connection is established between the mobile device (101) and the assistive device (103) based on the access link, the usage permission, and the validity duration. The mobile device (101) captures measurement data from the sensors embedded in the assistive device (103) based on the secure wireless connection. Furthermore, the mobile device (101) simultaneously records video data of the user wearing the assistive device (103) while capturing the measurement data from the plurality of sensors embedded in the assistive device (103). The mobile device (101) is equipped with advanced processing capabilities to handle data encryption and decryption, ensuring that all captured data remains secure and private.

The first mode of communication is one of a Short Message Service (SMS), an email application, a push notification, in-app messaging, cloud-enabled VoIP, a chat application, and others. The second mode of communication is one of a Bluetooth connection, a Wi-Fi connection, and any other short-range mode of communication. The mobile device (101) determines whether the access link is valid based on the unique identifier included in the recording message, the usage permission, and the validity duration. Further, the mobile device (101) establishes a secure wireless connection between the mobile device (101) and the assistive device (103) worn by the user (108) based on the secure time-bound link when the access link is valid. The mobile device (101) sets the recording mode as anonymous at the assistive device based on a recording type indicated in the access link and anonymously captures measurement data from the plurality of sensors embedded in the assistive device (103) based on the secure wireless connection. Simultaneously, the mobile device (101) points the camera associated with the mobile device (101) towards the user wearing the assistive device (103) and records the video data of the user wearing the assistive device (103) using the camera of the mobile device (101). Further, the mobile device (101) determines an expiry of at least one of the validity duration and the usage permission received in the recording message and automatically terminates the secure wireless connection between the mobile device (101) and the assistive device (103). The mobile device's user interface is designed to be intuitive and user-friendly, allowing users to easily navigate through the system's features and settings.

The assistive device (103) is the device embedded with the sensors designed to improve patient care, monitor health conditions, and assist individuals with disabilities or specific medical needs. The assistive device (103) may include but is not limited to an orthotic device, a prosthetic device, a smart device, or any other mobility-assistive device designed to support or enhance the user's physical functionality. The assistive device (103) can be equipped with adaptive control systems that adjust the level of assistance based on real-time feedback from the sensors. The assistive device (103) can be customized or configured to suit the specific needs of the user (108), such as providing stability, aiding in movement, or compensating for a physical limitation. Customization options may include adjustable components, modular designs, and software configurations that tailor the device's functionality to the user's unique gait characteristics and rehabilitation goals. The assistive device (103) collects the measurement data through sensors, processes it, and sends it to the mobile device (101) for real-time monitoring, diagnosis, and intervention. In an embodiment, the assistive device (103) tracks, measures, and reports health data to the mobile device (101). These assistive devices (103) help healthcare professionals monitor patients' conditions, make informed decisions, and adjust treatments as necessary. These devices are used for managing chronic conditions, post-surgical recovery, or acute medical events. The assistive device's sensors are calibrated to ensure high accuracy and precision in data collection, providing reliable information for clinical assessments.

In an embodiment, the assistive devices (103) are associated with the microprocessor integrated with the sensors to collect various types of data that help in monitoring, improving functionality, and ensuring proper fit and performance. The sensors can measure biomechanical data, environmental factors, and device-specific performance metrics. The sensors include but are not limited to pressure sensors that track how much pressure is being placed on different parts of the device, temperature sensors for monitoring the temperature, motion and gait analysis sensors for monitoring the user's gait, walking speed, and posture. Further, the assistive device includes accelerometers to measure the acceleration in multiple directions, gyroscopes to measure angular velocity and orientation, Electromyography sensors (EMG) sensors to measure the electrical activity in the muscles, and Inertial Measurement Unit (IMU) that combine accelerometers, gyroscopes, and magnetometers to track motion and orientation. Additionally, the sensors include force sensors such as load cells, in-shoe pressure sensors, or force plates to measure ground reaction forces and pressure distribution, external sensors like cameras, optoelectronic sensors, and force plates, and additional sensor types including ultrasonic, radar, and GPS. Furthermore, the sensors include motion capture systems, both marker-based and markerless, are used to capture joint kinematics, while goniometers or inclinometers measure joint angles. Magnetometers assist in orientation sensing, and pressure and shear sensors analyze interface pressures between the prosthetic or orthotic device and the residual limb. Additionally, environmental sensors may detect external factors like terrain or slope, while wearable sensors embedded in smart textiles can monitor distributed forces, pressures, or limb health. Sensor fusion mechanisms integrate data from these sources for detailed and accurate gait analysis. These mechanisms employ machine learning techniques to adaptively improve the accuracy of gait assessments over time. The assistive device's data processing capabilities are enhanced by embedded microcontrollers that perform real-time analysis and decision-making, enabling dynamic adjustments to the device's operation.

In an embodiment, the assistive device (103) equipped with the sensors collects real-time biomechanical data, environmental factors, and device-specific performance metrics of the user(108). These sensors are strategically placed on the assistive device (103) to capture measurement data on the user's biomechanics, including limb acceleration, angular velocity, and orientation. The measurement data include biomedical and movement data like joint angles, movement patterns, step count, walking speed, stride length, balance data, and activity levels. Further, the measurement data include pressure exerted on the residual limb or body, force distribution across the foot or other body parts, load applied to joints or other critical areas, and others. Additionally, the measurement data include temperature readings from the residual limb or under the orthotic device to monitor skin health and prevent discomfort or injury due to excessive heat buildup. The measurement data also includes tactile or touch feedback from the capacitive sensors, piezoelectric sensors, or force sensors, which help the user (108) to gauge interactions with objects such as grasping or pressure application. Further, the assistive devices that include Surface electromyography (sEMG) sensors detect electrical signals from the user's muscles, which helps to measure muscle contraction strength. Simultaneously, the mobile device (101) captures the video data of the user (108) wearing the assistive device (103) while capturing the measurement data from the plurality of sensors embedded in the assistive device (103). The video data is recorded at high frame rates to ensure detailed motion capture, and is used for analyzing rapid movements or subtle deviations. A critical component of the system (100) is the precise synchronization of the measurement data and the video data, ensuring accurate correlation between movement patterns and visual observations. This synchronization is achieved through a common time reference, such as a GPS-based timestamp or a network time protocol, enabling detailed analysis of performance metrics of the user (108). The system's data synchronization techniques are designed to minimize latency and ensure that all data streams are accurately aligned, providing a comprehensive view of the user's performance.

While Fig 1a illustrates the hardware components of the system (100) for anonymous secure data capture, alternative embodiments may include different or additional components. The labels or names of these elements are illustrative and do not limit the invention's scope. Components may also be combined to perform similar functions. The system's architecture is flexible, allowing for the integration of emerging technologies or new sensor types as they become available. This adaptability ensures that the system remains at the forefront of technological advancements, providing users with the most up-to-date tools for data capture and analysis. The system's design also considers future scalability, with provisions for expanding the network to accommodate additional users, devices, or data sources without compromising performance or security.

Fig 1b is a block diagram that illustrates the hardware features of the mobile device according to the embodiments as disclosed herein.. With reference to Fig 1b, the mobile device (101) can encompass a diverse range of devices, including but not limited to mobile devices, smartphones, Personal Digital Assistants (PDAs), laptops, desktops, digital cameras and tablets. In an embodiment, the mobile device (101) includes a memory (104), an anonymous secure data controller (106), a processor (105), and an I/O interface (107).

The memory (104) stores instructions to be executed by the processor (105). The memory (104) can include non-volatile storage elements. Examples of such non-volatile storage elements may include magnetic hard disks, optical disks, floppy disks, flash memories, or forms of electrically programmable memories (EPROM) or electrically erasable and programmable (EEPROM) memories. In addition, the memory (104) may, in some examples, be considered a non-transitory storage medium. The term non-transitory may indicate that the storage medium is not embodied in a carrier wave or a propagated signal. However, the term non-transitory should not be interpreted to mean that the memory (104) is non-movable. In some examples, the memory (104) stores larger amounts of information. In certain examples, a non-transitory storage medium may store data that can over time change (e.g., in Random Access Memory (RAM) or cache). The memory (104) facilitates the interaction between the mobile device (101) and the assistive device (103) and stores temporary data during active communication with the central server (102).

The processor (105) may include one or a plurality of processors. The one or the plurality of processors is a general-purpose processor such as a central processing unit (CPU), an application processor (AP), or the like, a graphics-only processing unit such as a graphics processing unit (GPU), a visual processing unit (VPU), and/or an AI-dedicated processor such as a neural processing unit (NPU). The processor (105) may include multiple cores and is configured to execute the instructions stored in the memory (104). The processor (105) fetches and executes instructions from software and coordinates the activities of various hardware components. The processor (105) controls and manages data flow between different hardware components of the mobile device (101), such as the memory (RAM storage), sensors, camera, user interface, and input devices, and the assistive device (103).

The I/O interface (107) transmits the information between the memory (104) and external peripheral devices. The peripheral devices are the input-output devices associated with the assistive devices (103). The I/O interface (107) receives several pieces of information from a plurality of mobile device (101), assistive device (103), central servers (102), and the like. The I/O interface (107) ensures that the operating speed of the processor (105) is synchronized with respect to the input and output devices.

In an embodiment, the anonymous secure data controller (106) receives the recording message for anonymous secure data capture from the central server over a first mode of communication, where the recording message includes an access link that identifies the assistive device to be recorded, a usage permission indicating the number of permissible accesses, and a validity duration for the access link. The first mode of communication may utilize encryption protocols such as Transport Layer Security (TLS) or Secure Sockets Layer (SSL) to ensure the secure transmission of the recording message from the central server (102) to the anonymous secure data controller (106). Further, the anonymous secure data controller (106) displays the access link on a user interface of the mobile device (101) and detects the user interaction with the access link displayed on the user interface of the mobile device (101). The user interface is designed to provide visual cues or notifications to prompt the user to interact with the access link, ensuring a seamless user experience. Further, the anonymous secure data controller (106) establishes a secure wireless connection between the mobile device (101) and the assistive device (103) worn by the user (108) using a second mode of communication different from the first mode of communication in response to receiving the user interaction. The secure wireless connection is established between the mobile device (101) and the assistive device (103) based on the access link, the usage permission, and the validity duration. The anonymous secure data controller (106) captures measurement data from sensors embedded in the assistive device (103) based on the secure wireless connection. These sensors may include accelerometers, gyroscopes, or biometric sensors that provide real-time data on the user's movements or physiological parameters. Simultaneously, the anonymous secure data controller (106) records the video data of the user (108) wearing the assistive device (103) while capturing the measurement data from the plurality of sensors embedded in the assistive device (103). The video data is encoded using efficient codecs such as H.264 or H.265 to optimize storage and transmission. Further, the anonymous secure data controller (106) transmits the video data of the user (108) wearing the assistive device (103) and the measurement data from the plurality of sensors embedded in the assistive device (103) to the central server (102). The transmission may occur over a secure channel, employing end-to-end encryption to protect the integrity and confidentiality of the data.

In an embodiment, the first mode of communication is one of a Short Message Service (SMS) or an email application. The SMS or email may contain a hyperlink that, when clicked, automatically launches the application on the mobile device (101) to initiate the secure data capture process. The second mode of communication is one of a Bluetooth connection, a Wi-Fi connection, or any other short-range mode of communication. Bluetooth Low Energy (BLE) is used to minimize power consumption while maintaining a reliable connection between the devices. Further, the access link includes a unique identifier for validation of the access link, an assistive device identifier, and a recording type as anonymous. The unique identifier is a cryptographic token that ensures the authenticity of the access link and prevents unauthorized access.

In an embodiment, the secure wireless connection between the mobile device (101) and the assistive device (103) worn by the user (108) includes determining, by the anonymous secure data controller (106), whether the access link is valid based on the unique identifier included in the recording message, the usage permission, and the validity duration. The validation process may involve querying a remote authentication server to verify the credentials associated with the access link. Further, the anonymous secure data controller (106) establishes a secure wireless connection between the mobile device (101) and the assistive device (103) worn by the user (108). The connection is established using a handshake protocol that negotiates encryption keys and communication parameters to ensure a secure and reliable link.

In an embodiment, the anonymous secure data controller (106) sets a recording mode as anonymous at the assistive device (103) based on a recording type indicated in the access link. The recording mode may configure the assistive device (103) to mask or obfuscate any personally identifiable information (PII) during data capture. Further, the anonymous secure data controller (106) anonymously captures the measurement data from the plurality of sensors embedded in the assistive device (103) based on the secure wireless connection. The data is anonymized by aggregating or transforming it into a format that prevents the identification of individual users.

In an embodiment, the anonymous secure data controller (106) starts a camera (109) of the mobile device (101). The camera (109) is pointed towards the user (108) wearing the assistive device (103). The camera (109) is equipped with image stabilization and autofocus features to ensure high-quality video capture. Further, the anonymous secure data controller (106) records the video data of the user (108) wearing the assistive device (103) using the camera (109) of the mobile device (101). The video data is stored temporarily on the mobile device (101) before being transmitted to the central server (102) for further processing and analysis.

In an embodiment, the anonymous secure data controller (106) determines an expiry of at least one of the validity duration and the usage permission received in the recording message and automatically terminates the secure wireless connection between the mobile device (101) and the assistive device (103). The termination process may involve sending a disconnect signal to the assistive device (103), ensuring that no further data is captured or transmitted beyond the authorized period. Additionally, the anonymous secure data controller (106) may log the disconnection event and notify the user through the mobile device interface, providing a summary of the data capture session.

While Fig. 1b illustrates the hardware components of the mobile device (101), alternative embodiments may include different or additional components. The labels or names of these elements are illustrative and do not limit the invention's scope. Components may also be combined to perform similar functions.

Fig. 1c is a block diagram that illustrates the hardware features of the central server, according to the embodiments as disclosed herein. The central server (102) includes a memory (110), a processor (111), an anonymous secure data controller (112) and an I/O interface (113).

In an embodiment, the central server (102) receives a request message from the mobile device (101) for anonymous secure data capture from the assistive device (103) and generates the recording message for the anonymous secure data capture based on a ULID Specification in response to receiving the request message. The recording message includes the access link that identifies the assistive device to be recorded, the usage permission indicating the number of permissible accesses, and the validity duration for the access link. The central server (102) further transmits the recording message for the anonymous secure data capture to the mobile device (101) using the first mode of communication. The request message includes at least one of a usage permission request and a validity duration request. The central server's communication protocols are designed to minimize latency and ensure that data capture requests are processed in real-time, providing users with immediate feedback and access to the system's features.

While Fig 1c illustrates the hardware components of the central server (102) for anonymous secure data capture, alternative embodiments may include different or additional components. The labels or names of these elements are illustrative and do not limit the invention's scope. Components may also be combined to perform similar functions. The system's architecture is flexible, allowing for the integration of emerging technologies or new sensor types as they become available. This adaptability ensures that the system remains at the forefront of technological advancements, providing users with the most up-to-date tools for data capture and analysis. The system's design also considers future scalability, with provisions for expanding the network to accommodate additional users, devices, or data sources without compromising performance or security.

FIG 2 is a flow diagram that illustrates anonymous secure data capture from the assistive device (103) worn by the user (108) according to the embodiments as disclosed herein. The figure describes the proposed solution that focuses on utilizing the mobile device (101) with a dedicated application connected to the internet within proximity of the assistive device (103). The mobile device (101) is cloud-based for patient security and backup, enabling safe updates. The cloud infrastructure is designed to support high availability and redundancy, ensuring that data is not lost even in the event of a device failure. The mobile application is equipped with end-to-end encryption protocols to protect data during transmission and storage. Additionally, the application can perform real-time data analysis and provide feedback to the user, enhancing the overall functionality of the assistive device.

At step S201, the central server receives a request message from the user through the mobile device (101) for anonymous secure data capture from the assistive device (103). The mobile device (101), after establishing a communication network with the central server (102), transmits a request message using the network protocols. The central server (102), after verifying the credentials and necessary permissions for data transmission, employs a multi-factor authentication process to ensure that only authorized devices can initiate data capture. This process may include biometric verification or a one-time password sent to the user's registered contact method. The server also logs each request for auditing purposes, maintaining a detailed record of all interactions for security and compliance.

At step S202, the central server (102) generates the recording message for the anonymous secure data capture based on a Universally Unique Lexicographically Sortable Identifier (ULID) Specification in response to receiving the request message. The ULID specification defines a type of unique identifier that can be used in distributed systems for a wide range of applications. The ULIDs are lexicographically sortable, time-based, and compact. The time-based component ensures that the ULID is unique not only by randomness but also by time. The time-based properties of ULIDs can offer several advantages that enhance the security of systems and applications. The time-sensitive nature of ULIDs, specifically the fact that they are lexicographically sortable and include a timestamp component, can be leveraged in various ways to improve both security and efficiency in assistive devices (103). When a ULID is used for session IDs, transaction IDs, or token-based authentication, the timestamp allows the central server to check whether a particular request is too old (e.g., a request with a timestamp older than a certain threshold). This prevents replay attacks where the attacker sends a previously valid request that the system would mistakenly accept. Furthermore, the ULID's compact size reduces the overhead in data transmission, making it ideal for low-bandwidth environments.

In an embodiment, the recording message includes the access link that identifies the assistive device (103) to be recorded, the usage permission indicating the number of permissible accesses, and the validity duration for the access link. Sharing information about the number of permissible accesses from the central server to the mobile device (101) improves security, performance, and resource management. Rate limiting prevents brute force or credential stuffing attacks, where attackers try multiple passwords or keys to gain unauthorized access. If each device has a limited number of attempts to access a service, the attack's success is severely reduced. By sharing information about permissible accesses, systems can identify anomalous or potentially malicious behavior. Additionally, the access link is encrypted using a public key infrastructure (PKI) to ensure that only the intended recipient can decrypt and use it, further enhancing security.

Further, at step S203, the central server (102) transmits the recording message, which includes the usage permission indicating the number of permissible accesses and the validity duration for the access link, to the mobile device (101). The transmission is secured using Transport Layer Security (TLS) to prevent eavesdropping and tampering during data exchange. The central server (102) also includes a digital signature with the recording message to verify its authenticity and integrity upon receipt by the mobile device (101). This ensures that the message has not been altered in transit and originates from a trusted source.

At step S204, the mobile device (101) receives the recording message for anonymous secure data capture from the central server (102) over the first communication link established earlier. The central server might send the recording message to the mobile device (101) as an SMS, email, or application chat application. Further, the recording message includes the access link that identifies the assistive device (103) to be recorded, a usage permission indicating the number of permissible accesses, and a validity duration for the access link. The mobile device (101) processes the message using its built-in security module, which verifies the digital signature and decrypts the access link using its private key. This ensures that the message is both authentic and confidential, preventing unauthorized access to sensitive information.

At step S205, the mobile device (101) displays the access link, which includes a unique identifier for validation of the access link, an assistive device identifier, and a recording type as anonymous on a user interface of the mobile device of the user (108). The unique identifier helps in the identification of the validation of the access link, whereas the assistive device identifier helps in the identification of the particular assistive device (103) from which the data stream needs to be collected. Further, setting the recording type to be anonymous helps in securing the data as the mobile device (101) does not have access to the information received from the assistive device (103). The user interface is designed to be intuitive, providing clear instructions and feedback to the user to facilitate seamless interaction. Additionally, the application may offer customization options, allowing users to adjust privacy settings and data capture preferences according to their needs.

At step S206, the mobile device (101) verifies if the user interaction with the access link is displayed on the user interface of the mobile device (101). If the user interaction with the access link is not displayed on the user interface of the mobile device (101), the mobile device (101) initiates the process of displaying as illustrated in the figure. The mobile device (101) employs a notification system to alert the user of the pending interaction, ensuring that the access link is promptly addressed. This system may include visual, auditory, or haptic feedback to accommodate different user preferences and accessibility needs.

If the user interaction with the access link is displayed at step S206, the mobile device (101) determines whether the access link is valid based on the unique identifier included in the recording message, the usage permission, and the validity duration as illustrated at step S207. If the access link is not valid, the mobile device (101) requests the central server (102) to share the access link and receives the recording message for anonymous secure data capture from a central server (102) over the first mode of communication. The mobile device (101) uses a secure handshake protocol to request a new access link, ensuring that the communication remains confidential and tamper-proof. The server may also implement a rate-limiting mechanism to prevent abuse of the access link request process.

At step S208, if the access link is valid, the mobile device (101) establishes a secure wireless connection with the assistive device (103) worn by the user (108) using a second mode of communication, which includes Bluetooth Low Energy (BLE), Wi-Fi connection, Near Field Communication (NFC), ZigBee, Ultra-Wideband (UWB), and other short-range communication protocols. At first, the mobile device (101) scans for the nearby connectable devices. Once the desired assistive device is found, the mobile device (101) initiates pairing where cryptographic keys are exchanged to secure the connection. After successful pairing, a connection request is sent by the mobile device (101), and the assistive device (103) responds to establish a secure wireless connection. The pairing process is designed to be user-friendly, with automatic detection and connection features to minimize user intervention. Additionally, the system supports dynamic key exchange, allowing for periodic key updates to maintain a high level of security throughout the connection.

At step S209, the mobile device (101) sets a recording mode as anonymous at the assistive device (103) based on the recording type indicated in the access link. Setting the recording mode as anonymous reduces the risk of targeted attacks such as phishing, hacking, or man-in-the-middle attacks since the source and destination remain concealed. The assistive device (103) is programmed to operate in a low-power mode during data capture, optimizing battery life while maintaining performance. The device's firmware is regularly updated to incorporate the latest security patches and enhancements, ensuring ongoing protection against emerging threats.

Further, after the successful establishment of the second mode of communication, the measurement data from the sensors embedded in the assistive device (103) are exchanged through the secure wireless connection. The camera (109) associated with the mobile device (101) is pointed towards the user (108) wearing the assistive device (103), and simultaneously, the mobile device (101) records the video data of the user (108) wearing the assistive device (103) while capturing the measurement data from the plurality of sensors embedded in the assistive device (103) as illustrated at step S210. The sensors may include accelerometers, gyroscopes, heart rate monitors, and other biometric sensors, providing comprehensive data for analysis. The video data is processed using advanced techniques to extract relevant features and patterns, which can be used to enhance the functionality of the assistive device.

At step S211, the mobile device (101) transmits the measurement data collected from the sensors and the video data of the user (108) wearing the assistive device (103) through the first mode of communication. Further, at step S212, the mobile device (101) verifies the validity of the access link. On determining the expiry of the validity duration or the usage permission received in the recording message, the mobile device (101) automatically terminates the secure wireless connection between the mobile device (101) and the assistive device (103) as illustrated at step S213. The termination process is designed to be seamless, ensuring that no data is lost or corrupted during disconnection. The mobile device (101) also logs the session details, including the duration and data volume, for future reference and analysis.

Fig 3 illustrates a use case scenario of the anonymous secure data capture from the assistive devices according to the embodiments as disclosed herein. The figure illustrates the scenario where the clinician requests for the data to be captured from the patient through the central server and obtains the required data securely from the assistive device (103). The assistive device (103) is equipped with multiple sensors, such as accelerometers, gyroscopes, and heart rate monitors, which continuously collect physiological and movement data. The central server (102) employs advanced encryption protocols to ensure that the data remains secure and anonymous during transmission. Additionally, the system (100) is designed to comply with healthcare regulations such as HIPAA, ensuring that patient privacy is maintained throughout the data capture and transmission process.

At step S301, the clinician transmits a request message to the central server (102) requesting for the data to be captured from the assistive device (103). As a result, the central server (102) generates the recording message, which includes an access link which is temporary or time bound that identifies the assistive device (103) to be recorded, a usage permission indicating the number of permissible accesses, and a validity duration for the access link is transmitted to the mobile device (101) of the user (108) as illustrated at step S302. The recording message is encrypted using a public key infrastructure (PKI) to ensure that only authorized devices can decrypt and access the information. The access link is uniquely generated for each request, preventing unauthorized reuse or interception by malicious entities. The central server (102) logs each request and access attempt, providing an audit trail for security and compliance purposes.

At step S303, the generated recording message is transmitted through Email or SMS to the mobile device (101) of the user (108) from the central server (102). Further, the mobile device (101) receives the recording message as illustrated at step S304. The access link is displayed on the user interface of the mobile device (101). The mobile device (101) detects if the link is displayed on the user interface of the mobile device (101). If the application is installed on the device, clicking the link will launch the application directly. If the application is not installed, clicking the link by the user (108) or the caretaker or clinician, will start downloading the application, and once downloaded, the mobile device (101) will automatically open the application. The application is designed to be lightweight and compatible with various operating systems, ensuring broad accessibility. It also includes a user-friendly interface that guides the user through the data capture process, minimizing the potential for user error.

At step S306, the mobile device (101) verifies whether the access link is valid based on the unique identifier included in the recording message, the usage permission, and the validity duration. On determining the validity of the access link, the mobile device (101) establishes the secure wireless connection using BLE between the mobile device (101) and the assistive device (103) worn by the user (108) as illustrated in step S307a. The BLE connection is encrypted using AES-128 encryption, providing a secure channel for data transmission. The mobile device (101) continuously monitors the connection quality and automatically re-establishes the link if any disruptions occur, ensuring uninterrupted data capture.

Further, at step S308, the mobile device (101) starts to capture measurement data from the sensors embedded in the assistive device (103). Simultaneously, the user (108) or the caretaker points the camera (109) of the mobile device (101) towards the user (108) wearing the assistive device (103) and starts to record the video data while capturing the measurement data. Additionally, the user (108) carries out the activity while recording the video data as shown in step S310. The application synchronizes the sensor data with the video feed, providing a comprehensive view of the user's activity and physiological responses. The video data is compressed in real-time to reduce file size without compromising quality, facilitating efficient storage and transmission.

At step S311, the mobile device (101) determines the expiry of one of the validity duration and the usage permission received in the recording message. On determining the expiry of the validity duration and the usage permission received in the recording message, the mobile device (101) stops recording the video data as illustrated in step S312. The application provides a notification to the user (108) or caretaker, indicating the end of the recording session. The system is designed to automatically save the captured data to prevent any loss due to unexpected interruptions or device malfunctions.

At step S313, the mobile device (101) automatically terminates the secure wireless connection between the mobile device (101) and the assistive device (103). Further, the mobile device (101) shares the video data of the user (108) wearing the assistive device (103) and the measurement data from the sensors embedded in the assistive device (103) to the central server (102) as shown in step S314. Step S314a illustrates the upload of the video data and the measurement data to the central server from the mobile device (101), after which the central server (102) receives the video data of the user (108) wearing the assistive device (103) and the measurement data from the sensors embedded in the assistive device (103) as illustrated at step S315. Further, the clinician accesses the video data of the user (108) wearing the assistive device (103) and the measurement data captured from the sensors for monitoring. The central server (102) employs machine learning techniques to analyze the data, providing insights and alerts to the clinician regarding the user's health status. The clinician can access the data through a secure web portal, which offers visualization tools and reporting features to aid in patient assessment and decision-making.

Fig 4 illustrates a use case scenario of the anonymous secure data capture from the prosthetic device worn by a user according to the embodiments as disclosed herein. The figure illustrates a user (108) with a prosthetic limb wearing a smart prosthesis equipped with sensors that monitor various parameters like pressure, temperature, and movement. These sensors are strategically placed within the prosthetic limb to ensure accurate data capture, with pressure sensors embedded in the footbed to measure load distribution and temperature sensors integrated into the socket to monitor skin temperature. The user or the caretaker or the clinician, uses the application in the mobile device (101) to track the performance of the user's prosthetic, ensuring its proper fit, optimizing his walking pattern, and preventing injuries. The application provides real-time feedback and alerts, allowing for immediate adjustments to the prosthetic settings if abnormal readings are detected. The caretaker or the user (108) is authenticated to access the mobile device (101) for secure capture of the measurement data from the assistive device (103). The mobile device (101) of the user or the clinician or caretaker who has access to the central server (102) retrieves the time-synchronized recording message from the central server (102) and presents them through the application or the user interface. The application is designed to display the access link alongside the video data in a synchronized manner, allowing user (108) to seamlessly analyze the relationship between real-time movements and measurement data. This synchronization is achieved through a timestamping mechanism that aligns sensor data with video frames, providing a comprehensive view of the user's mobility.

At step S401, the mobile device (101) receives the recording message through the email for anonymous secure data capture from the central server (102) over the first mode of communication. The recording message includes the access link that identifies the assistive device (103) to be recorded, the usage permission indicating the number of permissible accesses, and the validity duration for the access link. The access link is generated using a secure hash technique to ensure its integrity and prevent tampering. The time-synchronized access link contains the current time from the central server (102), a unique identifier for the session, and the duration for which the access link is valid. This unique identifier is a cryptographic token that ensures the session's authenticity and prevents unauthorized access.

At step S402, the mobile device (101) displays the access link on the user interface of the mobile device (101) of the user or the caretaker who is authenticated to access the central server (102). The user interface is designed to be intuitive, with clear instructions guiding the user or caretaker through the process of accessing the data. Once the user or the caretaker clicks the access link displayed on the user interface of the mobile device (101), the application is opened. The application allows the user to connect to the assistive device (103) worn by the user. The connection process involves a secure handshake protocol that verifies the identity of both the mobile device and the assistive device, ensuring that data is exchanged only between trusted entities.

The step S404 illustrates the establishment of the wireless connection with the assistive device (103) via the short-range communication protocol such as BLE. The BLE protocol is chosen for its low power consumption and ability to maintain a stable connection over short distances, which is ideal for wearable devices. The assistive device (103) associated with the sensors produces the various parameters like pressure, temperature, measurement data indicating movements of the user (108), and others. These parameters are sampled at a high frequency to capture detailed information about the user's gait and activity levels, which are then processed by the mobile device to provide actionable insights.

At step S405, the mobile device (101) is pointed towards the user (108) and the camera (109) is activated to capture the video data of the user (108) wearing the assistive device (103). The camera (109) is equipped with image stabilization technology to ensure clear and steady video capture, even if the user (108) is moving. The figure illustrates a caretaker of the user capturing the video data of the user wearing the assistive device (103) from a fixed distance. This fixed distance is calibrated to provide an optimal field of view, capturing the full range of motion of the prosthetic limb.

Further at the step S406, the mobile device (101) receives measurement data like pressure data, gait and movement data (for example, a limp or uneven walking pattern), the temperature data (e.g., indicating possible overheating), and others from the sensors associated with the assistive device (103). The data is processed using advanced techniques that detect anomalies and provide recommendations for adjustments. For instance, if the temperature data indicates overheating, the system may suggest loosening the prosthetic socket or taking a break to prevent skin irritation.

At step S407, the mobile device (101) determines the expiry of the validity duration and the usage permission received in the recording message and automatically terminates the secure wireless connection between the mobile device (101) and the assistive device (103). This automatic termination maintains the security of the system, ensuring that the connection is not left open to potential unauthorized access. Further, the mobile device (101) transmits the measurement data received from the sensors associated with the assistive device (103) and the video data recording of the user wearing the assistive device (103) to the central server (102). The data transmission is performed over a secure channel, using end-to-end encryption to protect the data from interception during transit.

In an embodiment, the wireless connection link established between the mobile device (101) and the assistive device (103) is secured using encryption. Specifically, the connection is encrypted with a unique encryption key that is exclusively associated with the assistive device (103). This unique encryption key is generated using a public key infrastructure (PKI) system, which ensures that only authorized devices can decrypt the data. This unique encryption key ensures that data exchanged over the wireless connection remains confidential and protected from unauthorized access, thereby enhancing the security and integrity of the communication between the mobile device (101) and the assistive device (103). The encryption protocol used is compliant with industry standards, providing a robust defense against potential cyber threats.

The system (100) supports remote monitoring and collaboration through a web-based portal accessible to healthcare providers. The portal is designed with a user-friendly interface that allows healthcare providers to easily navigate through patient data and generate reports. Remote users can access patient data, review assistive device's data reports, and provide treatment recommendations. This feature is particularly valuable for telemedicine applications and the management of patients with mobility impairments. By combining wearable technology, advanced data processing, and cloud computing, the proposed system (100) offers a powerful tool for understanding and improving mobility. Its potential applications extend to various fields, including prosthetics, orthotics, rehabilitation, and sports performance analysis. Ensuring the security and privacy of patient data is paramount in remote monitoring systems. Robust encryption protocols are implemented to protect data both in transit and at rest. Access to patient data is strictly controlled, with appropriate role-based access permissions granted to healthcare providers and authorized personnel. Additionally, the system (100) adheres to relevant data protection regulations and industry standards to safeguard patient confidentiality. The system (100) is designed to be scalable, allowing for integration with other healthcare systems and expansion to accommodate a growing number of users and devices.

The foregoing description of the specific embodiments will so fully reveal the general nature of the embodiments herein that others can, by applying current knowledge, readily modify and or adapt for various applications such specific embodiments without departing from the generic concept, and, therefore, such adaptations and modifications are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

## Claims

1. A method for anonymous secure data capture from an assistive device (103) worn by a user (108), comprising:
receiving, by a mobile device (101), a recording message for anonymous secure data capture from a central server (102) over a first mode of communication, wherein the recording message comprises an access link that identifies the assistive device (103) to be recorded, a usage permission indicating number of permissible accesses, and a validity duration for the access link;
displaying, by the mobile device (101), the access link on a user interface of the mobile device (101);
detecting, by the mobile device (101), a user interaction with the access link displayed on a user interface of the mobile device (101);
establishing, by the mobile device (101), in response to receiving the user interaction, a secure wireless connection between the mobile device (101) and the assistive device (103) worn by the user (108) using a second mode of communication different from the first mode of communication, wherein the secure wireless connection is established between the mobile device (101) and the assistive device (103) based on the access link, the usage permission, and the validity duration;
capturing, by the mobile device (101), measurement data from a plurality of sensors associated with the assistive device (103) based on the secure wireless connection;
simultaneously recording, by the mobile device (101), a video data of the user (108) wearing the assistive device (103) while capturing the measurement data from the plurality of sensors associated with the assistive device (103); and
transmitting, by the mobile device (101), the video data of the user (108) wearing the assistive device (103) and the measurement data from the plurality of sensors associated with the assistive device (103) to the central server (102).

2. The method as claimed in claim 1, wherein the first mode of communication is one of a Short Message Service (SMS), an email application, and a chat application and/or the second mode of communication is one of a Bluetooth connection, a wi-fi connection and any other short range communication protocol.

3. The method as claimed in claim 1 or 2, wherein the access link comprises a unique identifier for validation of the access link, an assistive device identifier, and a recording type as anonymous, optionally
wherein establishing, by the mobile device (101), a secure wireless connection between the mobile device (101) and the assistive device (103) worn by the user (108) comprises:
determining, by the mobile device (101), whether the access link is valid based on the unique identifier included in the recording message, the usage permission and the validity duration; and
establishing, by the mobile device (101), a secure wireless connection between the mobile device (101) and the assistive device (103) worn by the user (108) based on the access link when the access link is valid.

4. The method as claimed in any preceding claim, wherein capturing, by the mobile device (101), measurement data from the plurality of sensors associated with the assistive device (103) based on the secure wireless connection comprises:
setting, by the mobile device (101), a recording mode as anonymous at the assistive device (103) based on a recording type indicated in the access link; and
anonymously capturing, by the mobile device (101), the measurement data from the plurality of sensors associated with the assistive device (103) based on the secure wireless connection, while recording the video data using the mobile device (101).

5. The method as claimed in any preceding claim, wherein recording, by the mobile device (101), a video data of the user (108) wearing the assistive device (103) at same time comprises:
starting, by the mobile device (101), a camera (109) of the mobile device (101), wherein the camera (109) is pointed towards the user (108) wearing the assistive device (103); and
recording, by the mobile device (101), the video data of the user (108) wearing the assistive device (103) using the camera (109) of the mobile device (101), while simultaneously receiving the measurement data from the assistive device (103).

6. The method as claimed in any preceding claim, comprising:
determining, by the mobile device (101), an expiry of at least one of the validity duration and the usage permission received in the recording message; and
automatically terminating, by the mobile device (101), the secure wireless connection between the mobile device (101) and the assistive device (103) based on the expiry of at least one of the validity duration and the usage permission received in the recording message.

7. The method as claimed in any preceding claim, comprising:
receiving, by the central server (102), a request message from the mobile device (101) for anonymous secure data capture from the assistive device (103), wherein the request message comprises at least one of a usage permission request and a validity duration request;
generating, by the central server (102), the recording message for the anonymous secure data capture based on a Universally Unique Lexicographically Sortable Identifier (ULID) Specification in response to receiving the request message, wherein the recording message comprises the access link that identifies the assistive device (103) to be recorded, the usage permission indicating number of permissible accesses, and the validity duration for the access link; and
transmitting, by the central server (102), the recording message for the anonymous secure data capture to the mobile device (101) using the first mode of communication.

8. A system (100) to anonymous secure data capture from an assistive device (103) worn by a user (108), comprising:
a central server (102);
a mobile device (101) of the user (108) connected to the central server (102); and
an assistive device (103) worm by the user (108), wherein the mobile device (101) comprises a memory (104) comprising an application, and an anonymous secure data controller (106) connected to the memory (104), wherein the anonymous secure data controller (106) configured to:
receive a recording message for anonymous secure data capture from a central server (102) over a first mode of communication, wherein the recording message comprises an access link that identifies the assistive device (103) to be recorded, a usage permission indicating number of permissible accesses, and a validity duration for the access link;
display the access link on a user (108) interface of the mobile device (101);
detect a user (108) interaction with the access link displayed on a user interface of the mobile device (101);
establish a secure wireless connection between the mobile device (101) and the assistive device (103) worn by the user (108) using a second mode of communication different from the first mode of communication, in response to receiving the user (108) input, wherein the secure wireless connection is established between the mobile device (101) and the assistive device (103) is established based on the access link, usage permission, and the validity duration;
capture measurement data from a plurality of sensors associated with the assistive device (103) through the secure wireless connection;
simultaneously record a video data of the user (108) wearing the assistive device (103) while capturing the measurement data from the plurality of sensors associated with the assistive device (103); and
transmit the video data of the user (108) wearing the assistive device (103) and the measurement data from the plurality of sensors associated with the assistive device (103) to the central server (102).

9. The system (100) as claimed in claim 8, wherein the first mode of communication is one of a Short Message Service (SMS), an email application, and a chat application, and wherein the second mode of communication is one of a Bluetooth connection, a wi-fi connection and any other short-range mode of communication.

10. The system (100) as claimed in claim 8 or 9, wherein the access link comprises a unique identifier for validation of the access link, an assistive device identifier, and a recording type as anonymous.

11. The system (100) as claimed in any one of claims 8 to 10, wherein establish the secure wireless connection between the mobile device (101) and the assistive device (103) worn by the user (108) comprises:
determine whether the access link is valid based on included in the recording message, the usage permission and the validity duration; and
establish a secure wireless connection between the mobile device (101) and the assistive device (103) worn by the user (108) based on the access link when the access link is valid.

12. The system (100) as claimed in any one of claims 8 to 11, wherein capture the measurement data from the plurality of sensors associated with the assistive device (103) based on the secure wireless connection comprises:
set a recording mode as anonymous at the assistive device (103) based on a recording type indicated in the access link; and
anonymously capture the measurement data from the plurality of sensors associated with the assistive device (103) in the recording mode as anonymous based on the secure wireless connection, while recording the video data using the mobile device (101).

13. The system (100) as claimed in any one of claims 8 to 12, wherein record the video data of the user (108) wearing the assistive device (103) at same time comprises:
start the camera (109) of the mobile device (101), wherein the camera (109) is pointed towards the user (108) wearing the assistive device (103); and
record the video data of the user (108) wearing the assistive device (103) using the camera of the mobile device (101), while simultaneously receiving the measurement data from the assistive device (103).

14. The system (100) as claimed in any one of claims 8 to 13, wherein the anonymous secure data controller (106) is configured to:
determine an expiry of at least one of the validity duration and the usage permission received in the recording message; and
automatically terminate the secure wireless connection between the mobile device (101) and the assistive device (103).

15. The system (100) as claimed in any one of claims 8 to 14, wherein the central server (102) is configured to:
receive a request message from a mobile device (101) for anonymous secure data capture of the assistive device (103), wherein the request message comprises at least one of a usage permission request and a validity duration request;
generate a recording message for the anonymous secure data capture based on a ULID Specification in response to receiving the request message, wherein the recording message comprises an access link that identifies the assistive device (103) to be recorded, the usage permission indicating number of permissible accesses, and a validity duration for the access link; and
transmit the recording message for the anonymous secure data capture to the mobile device (101) using a first mode of communication, wherein the recording message comprises an access link that identifies the assistive device (103) to be recorded, a usage permission indicating number of permissible accesses, and a validity duration for the access link.
